# EUROPEAN PATENT APPLICATION

(11) **EP 2 335 645 A2**
(43) Date of publication of application: **22.06.2011**
(21) Application number: 10183047.9
(22) Date of filing: 28.03.2002
(51) Int. Cl.: A61F 2/00, A61B 17/04

(54) **Implant inserted without bone anchors**

(30) Priority: 29.03.2001 US 279794 P; 03.07.2001 US 302929 P; 25.07.2001 US 307836 P; 14.09.2001 US 322309 P; 25.03.2002 US 106086
(62) Divisional of application: 02721598.7
(71) Applicant: AMS Research Corporation, Minnetonka, MN 55343 (US)
(72) Inventor: Neisz, Johann J., Minnetonka, MN 55343 (US); Anderson, Kimberly A., Minnetonka, MN 55343 (US); Watschke, Brian P., Minnetonka, MN 55343 (US); Lund, Robert E., Minnetonka, MN 55343 (US); Gohman, James A., Minnetonka, MN 55343 (US)
(74) Representative: Vossius & Partner

(57) **Abstract**

The present invention discloses an implant for placement in the retropubic space of a patient. Novel methods and assemblies for use in conjunction with the implant are also described.

## Description

### Background

Loss of bladder control is a condition known as urinary incontinence. Millions of men and women of all ages suffer from this condition, which causes involuntary loss of urine. Although urinary incontinence may occur at any age, it is more common in women and in the elderly. Women may develop incontinence during pregnancy, childbirth or menopause. Older men may lose bladder control following prostate surgery. In addition to the medical aspects of this condition, the social implications for an incontinent patient include loss of self-esteem, embarrassment, restriction of social and sexual activities, isolation, depression and, in some instances, dependence on caregivers.

Continence problems may occur when the muscles of the urinary system malfunction or are weakened. Other factors, such as trauma to the urethral area, neurological injury, hormonal imbalance or medication side-effects, may also cause or contribute to incontinence problems.

In general, there are five basic types of incontinence: stress incontinence, urge incontinence, mixed incontinence, overflow incontinence and functional incontinence. Stress urinary incontinence (SUI) is the involuntary loss of urine that occurs due to sudden increases in intra-abdominal pressure resulting from activities such as coughing, sneezing, lifting, straining, exercise and, in severe cases, even simply changing body position. This condition usually occurs when the sphincter or pelvic muscles are weakened by, for example, childbirth or surgery.

Urge incontinence, also termed "hyperactive bladder," "frequency/urgency syndrome" or "irritable bladder," occurs when an individual experiences the immediate need to urinate and loses bladder control. Urge incontinence is a common problem that increases with advancing age or results from a kidney or bladder infection.

Mixed incontinence is the most common form of urinary incontinence. Mixed incontinence is a combination of the symptoms for both stress and urge incontinence. Overflow incontinence is a constant dripping or leakage of urine caused by an overfilled bladder. This condition often occurs in men due to the prevalence of obstructive prostate gland enlargement or tumor. Functional incontinence results when a person has difficulty moving from one place to another. It is generally caused by factors outside the lower urinary tract, such as deficits in physical function and/or cognitive function.

A variety of treatment options are currently available to treat incontinence. Some of these treatment options include external devices, indwelling catheters, behavioral therapy (such as biofeedback, electrical stimulation, or Kegal exercises), injectable materials, prosthetic devices and/or surgery. Surgical procedures can be used to completely restore continence in some instances.

Surgical procedures include sling procedures, colposuspension procedures, and needle suspension procedures. Colposuspension procedures seek to place the urethra in high retropubic position. The Marshall-Marchetti-Krantz procedure and the Burch procedure are examples of colposuspension procedures. The Marshall-Marchetti-Krantz procedure places sutures at the urethrovesical junction to the periosteum of the pubic bone. See Marshall et al., The Correction of Stress incontinence By Simple Vesicourethral Suspension; Surg. Gynecol. Obstet. Vol. 88, Pps. 509-518 (1949).

With the Burch procedure, sutures are placed at the urethrovesical junction to Cooper's ligament. See Gilj a et al., A Modified Raz Bladder Neck Suspension Operation (Transvaginal Burch), J. of Urol. Vol. 153, Pps. 1455-1457 (May 1995). A significant abdominal incision is associated with the Marshall-Marchetti-Krantz procedure. The Burch procedure has been performed abdominally, vaginally and laparoscopically. See Burch, Urethrovaginal Fixation to Cooper's Ligament for Correction of Stress Incontinence, Cystocele, and Prolapse, Am. J. Obst. & Gynecology, vol. 81 (No. 2), Pps. 281-290 (Feb. 1961); and Das et al., Laparoscopic Colpo-Suspension, J. of Urology, vol. 154, Pp. 1119-1121 (1995).

Needle suspension procedures elevate the urethra retropubically. They include Pereyra, Stamey, Raz, Gittes, Muszani and Vesica procedures. These procedures (except the Vesica procedure) place sutures transvaginally at the urethrovesical junction and are sutured to the abdominal wall through two small abdominal incisions. See Stamey, Endoscopic Suspension of the Vesical Neck for Urinary Incontinence in Females, Ann. Surgery, pp. 465-471, October 1980; Pereyra, A Simplified Surgical Procedure for the Correction o Stress Incontinence in Women, West. J. Surg., Obstetrics & Gynecology, pp. 243-246, July-August 1959; Holschneider et al., A Modified Pereyra Procedure In Recurrent Stress Urinary Incontinence: A 15-Year Review, Obstetrics & Gynecology, vol. 83, No. 4 Pps. 573-578 (1994). The Vesica procedure includes an abdominal incision where bone anchors are driven into the top of the pubic bone and sutures attached to the bone anchors are placed at the urethrovesical junction.

The first sling procedure was the Goebel-Stoeckel-Frannenheim procedure. The sling was autologous fascia that was placed beneath the urethra and suspended by sutures attached to the rectus fascia of the abdominal wall.

There are two general types of sling procedures. The first type of sling procedure utilizes bone screws and associated sutures to anchor a sling (e.g. on a posterior portion of the pubic bone). A commercial example of a bone screw sling procedure is a surgical procedure that utilizes the In-Fast Sling System, available from American Medical Systems of Minnetonka, Minnesota.

The second type of sling procedure is a minimally invasive surgical method involving the placement (e.g. by the use of a Stamey needle or other ligature carrier) of a sling to stabilize or support the bladder neck or urethra. See Horbach et al., A Suburethral Sling Procedure With Polytetrafluoroethylene For the Treatment of Genuine Stress Incontinence In Patients With Low Urethral Closure Pressure, J. Obstetrics & Gynecology, vol. 71, No. 4, Pps. 648-652 (April 1998); and Morgan et al., The Marled Sling Operation For the Treatment of Recurrent Stress Urinary Incontinence: A 16 Year Review, Am. J. Obstet. Gynecol., vol. 151, No. 2, Pps. 224-227, (Jan. 1985).

The slings described above differ in the type of material, sutures and points of anchoring based on the procedure being performed. In some cases, the sling is placed under the bladder neck and secured via suspension means (such as bone anchors or screws) through a vaginal incision. Bone anchors or screws raise the specter of bone infection, necrosis and other complications, although such complications are rare.

The second type of sling procedure (pubovaginal sling procedures that do not include bone anchors) anchor slings in the abdominal or rectus fascia. These types of procedures involve puncturing the abdominal wall of the patient to pass a needle. Complications associated with sling procedures are rare, but they include urethral obstruction, infection, development of de novo urge incontinence, bladder perforation, hemorrhage, prolonged urinary retention, and damage to surrounding tissue (e.g. caused by sling erosion). The likelihood of complications due to abdominal incisions varies and depends on the particular surgical procedure.

The TVT Tension-free Vaginal Tape procedure is a known sling procedure used in the United States. During the procedure, incisions are made in the abdominal (i.e. suprapubic) area and in the vaginal wall. Two curved, needle-like elements are connected at an end, to tension-free vaginal sling tape. A tape-free end of one of the needle-like elements is inserted through the vaginal incision and into the paraurethral space. Using a handle attached to the needle, the needle is angulated laterally (for example, to the right) to perforate the endopelvic fascia, guided through the retropubic space and passed through the abdominal incision. The handle is disconnected and the needle is then withdrawn through the abdominal wall, thereby threading a portion of the tape through the tissue of the patient. This technique is repeated with the other needle on the other side (for example, to the left), so that the tape is looped beneath the bladder neck or urethra. The tape is adjusted to provide appropriate support to the bladder neck or urethra. The tape ends are then cut at the abdominal wall leaving the ends of the sling anchored in the abdominal (rectus) fascia.

Complications associated with the TVT procedure include injury to blood vessels of the pelvic sidewall and abdominal wall, hematomas, urinary retention, and bladder and bowel injury. One serious disadvantage of the TVT procedure, particularly for surgeons unfamiliar with the surgical method, is the lack of information concerning the precise location of the needle tip relative to adjacent pelvic anatomy. A cadaver study indicated that the TVT needle is placed in close proximity to sensitive tissue such as superficial epigastric vessels, inferior epigastric vessels, the external iliac vessel and the obturator. See, Walters, Mark D., Percutaneous Suburethral Slings: State of the Art, presented at the conference of the American Urogynecologic Society, Chicago (October 2001).

If the TVT needle tip is allowed to accidentally pass across the surface of any blood vessel, lymphatic duct, nerve, nerve bundle or organ, serious complications can arise. These shortcomings, attempts to address these shortcomings and other problems associated with the TVT procedure are disclosed in PCT publication nos. PCT WO 00/74613 and PCT WO 00/74594.

Examples of incontinence procedures are disclosed in U.S. Pat. Nos. 5,112,344; 5,611,515; 5,842,478; 5,860,425; 5,899,909; 6,039,686, 6,042,534 and 6,110,101.

### Brief Summary

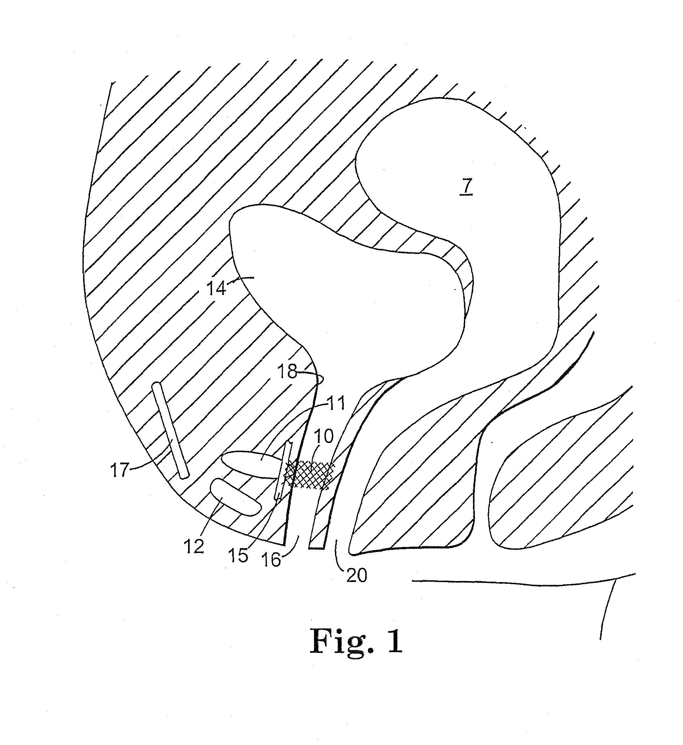

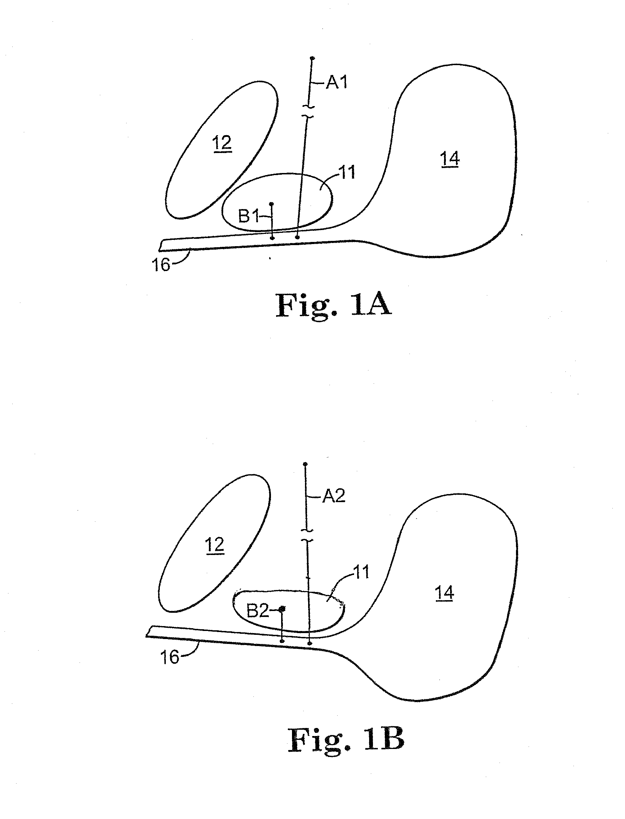

Fig. 1A schematically represents the position of anatomical structures such as the pubic bone 12, retropubic space 11, bladder 14 and urethra 16. The retropubic space 11 is a highly deformable cavity. It expands and collapses under the influence of surrounding tissue such as the bladder, etc. The relative positions of these structures or regions are shown at rest. In a healthy, continent individual, the external sphincter and other tissues and structures cooperate to resist flow of urine out of the bladder 14. In the rest or "non-stressed" condition, the distance between a midpoint of the retropubic space and an axial midpoint of the urethra 16 is B1. The distance between the axial midpoint of the urethra 16 and an upper, relatively fixed structure (for example, the rectus fascia, the top of the pubic bone or Cooper's ligament) is A1.

Fig. 1B schematically illustrates the effect of a stress event (e.g. coughing or sneezing) on the anatomical structures of Fig. 1A. There can be a marked descent of the bladder and urethra during certain types of stress events. The retropubic space 11 and its midpoint descend slightly. The distance A2 (between the relatively fixed structure and an axial midpoint of the urethra 16) is greater than the distance A1 (see Fig. 1A). The increase from A1 to A2 is more than the increase from B1 to B2. Healthy, continent individuals can nonetheless retain urine as their support structure can continue to close the urethra 16. With many types of incontinence, however, the intraurethral pressure during the stress event rises above the support structure's ability to close the urethra, resulting in leakage.

There is a debate in the medical community concerning the precise mechanism responsible for the success of sling procedures. Some commentators believe that slings correct incontinence by providing a backstop effect (i.e. preventing the distance A2 from expanding beyond a limit). The present invention recognizes the possibility that continence may be restored by providing dynamic support (i.e. a sling that is not securely attached to a fixed anatomical reference point). The dynamic support and continence may be provided without the need for invasive procedures that secure a sling to a fixed reference (e.g. Cooper's ligament, the pubic bone or rectus fascia). As a result, it is believed that the present invention is much less invasive and risks far fewer complications than the prior art sling procedures.

As used herein, the term "retropubic space" means that region of the body that is posterior to the pubic bone (i.e. the region that is posterior to the pubic ramus and pubic symphysis). This is an area of loose connective tissue between the bladder with its related fascia and the pubis. It includes endopelvic fascia. The retropubic space extends upward to the rectus fascia, but does not include the suprapubic area with the rectus fascia itself. The retropubic space does not extend beyond the sacrum. The phrases "space ofRetzius" or "cave of Retzius" are also used to describe portions of the retropubic space.

Conventional procedures exclude the possibility of anchoring a sling solely in the retropubic space. The prior art procedures suture the sling to a bone screw, the bone itself or tough, fixed tissue such as Cooper's ligament (which is fixed relative to the pubic bone). Other prior art procedures extend the sling through abdominal incisions and anchor the sling in rectus fascia of the suprapubic area.

Some surgeons believe that the retropubic space does not offer a sufficiently robust foundation for anchoring a sling. For example, for conventional sling procedures that do not use bone anchors, surgeons will typically extend the sling into the rectus fascia to firmly anchor the sling. Some procedures even suture the sling to the rectus fascia.

The present invention recognizes that, when disturbed by an implantable material, the retropubic space will generate tough fibrous tissue, providing substantial holding power for an implant placed in that space. This body reaction can be exploited to help restore continence.

The present invention recognizes that an implantable article (e.g. a dynamic sling or hemi-sling) may be anchored to structure in the retropubic space, without the need of bone anchors and without the need to suture the implant to Cooper's ligament, the pubic bone or the tough rectus fascia. With the present invention, the implant may be anchored in the retropubic space (e.g. to endopelvic fascia) without the need to extend upward into the abdominus or rectus fascia. This avoids complications associated with invasive abdominal incisions.

As used herein, the phrase "endopelvic fascia" means tissue that covers the pelvic organs and surrounds vessels and nerves in the pelvic region (e.g. in the subperitoneal space). Endopelvic fascia includes collagen, elastin and smooth muscle. These structures surround and support the viscera in the pelvic cavity and extend from the pelvic floor to the rectus fascia and respiratory diaphragm. As used herein, endopelvic fascia can include pubocervical fascia and periurethral fascia. Endopelvic fascia is also referred to as visceral pelvic fascia.

Pubocervical fascia is a significant component of urethrovescial junction support. Pubocervical fascia is a sheet of thick fibrous tissue that is located on the vagina underneath the bladder. Pubocervical fascia is anterior vaginal fascia that fuses with vaginal tissue, providing a hammock for the urethra and bladder. Proximally, the pubocervical fascia attaches to the cervix; distally it extends beneath the urethra and fuses with the perineal membrane of the ureogenital triangle; and laterally, it is connected to the pelvic wall at the fascial white line (arcus tendineus fasciae pelvis).

The pubocervical fascia forms a horizontal platform that supports the bladder, and its anterior portion supports the urethra. With increased abdominal pressure, the lower urinary tract is forced inferiorly and compressed against the pubocervical fascia while this fascial layer displaces to a lesser degree because of its elastic suspensory characteristics.

The present invention is directed to methods of placing implants, hemi-slings, dynamic slings or other articles for treating incontinence that do not require abdominal incisions, or bone anchors. The present invention recognizes that it is not necessary to anchor a sling or other implantable article directly in bone or in the tough abdominal (rectus) fascia. As a result, the present invention is less invasive than conventional procedures and exhibits less potential for experiencing the complications associated with bone anchoring procedures.

Since surgical tools for this procedure need not extend through the abdominal wall, the present invention reduces the risk that vulnerable tissue (such as the bladder) will be damaged by a surgical instrument. The implant is preferably inserted through a vaginal incision that is preferably as small as possible. Other surgical routes such as transurethral and transperineal are also within the scope of the present invention. The present invention is particularly suitable for use with concomitant procedures such as a sacral colpopexy or pelvic floor repair. The present invention also preferably does not preclude subsequent surgeries.

In one aspect, the present invention comprises a method of treating incontinence in a patient comprising the steps of i) providing an implant capable of eliciting a foreign body response, the implantable material being sized and shaped to be placed in the patient's retropubic space without extending through the patient's rectus fascia, ii) placing the implant in the retropubic space without securing the implant to substantially fixed anatomical structures such as the patient's pubic bone, periosteum of the pubic bone, Cooper's ligament and rectus fascia; and iii) eliciting a foreign body response with the implantable material.

Preferably, the step of placing the implant in the retropubic space includes the step of associating the implant with the patient's endopelvic fascia to more closely mimic characteristics of endopelvic fascia of a continent individual. More preferably, the step of associating the implant with the patient's endopelvic fascia includes the step of anchoring the implant with a mechanical fastener or a tissue adhesive or foam. There are a variety of different novel techniques and articles that may be used to place an implant in the retropubic space.

In another aspect, the present invention can comprise the steps of i) providing an implant that is sized and shaped be implanted in the patient's retropubic space and that is capable of eliciting a foreign body response; and ii) placing the implant in the retropubic space in a therapeutically effective position relative to the patient's urethra without extending the implant to the patient's rectus fascia, without suturing the implant to the patient's Cooper's ligament, and without using bone anchors. Preferably, the step of placing the implant includes the step of anchoring a first end of the implant with endopelvic fascia on one side of the patient's urethra and anchoring a second end of the implant with endopelvic fascia on the other side of the patient's urethra. A therapeutically effective position may, for example, be mid-urethra.

A variety of surgical procedures are contemplated. For example, the method could include the steps of passing a deployable anchoring member with an associated suture through endopelvic fascia; deploying the anchoring member in endopelvic fascia, and securing the implant to the suture. In a preferred embodiment, the method includes the step of extending the implant from the endopelvic fascia on one side of the patient's urethra, underneath approximately the mid-urethra, and to the endopelvic fascia on the other side of the patient's urethra.

A variety of different surgical approaches are contemplated including approaches utilizing a vaginal incision, transurethral approaches and laparoscopic approaches. Treatments for male incontinence and fecal incontinence are also contemplated herein, with the attendant inclusion of a transperineal approach.

The present invention also contemplates a novel assembly of components for a surgical procedure designed to treat incontinence. The components are useful in placing an implant in a patient's retropubic space during a surgical procedure. In one embodiment, the assembly comprises at least one deployable member for associating the implant with endopelvic fascia; and an inserter. A variety of inserters and deployable members are contemplated.

In a preferred embodiment, the inserter includes a sheath, and a movable member within the sheath. The movable member is operatively associated with the deployable member to move the deployable member between i) a retracted position with the deployable member at least partially received within the sheath of the inserter, and ii) an extended position that is spaced more distally to a distal end of the sheath than in the retracted position. Movement of the movable member causes the deployable member to move from the retracted position toward the extended position. Rotational and linear movement embodiments are disclosed. Preferably, the inserter includes a tissue stop to resist penetration of the distal end of the inserter beyond a predetermined distance.

In one embodiment, the deployable member is capable of assuming a first orientation that affords at least partial receipt of the deployable member within the sheath of the inserter, and a second orientation that affords association between the deployable member and endopelvic fascia. The deployable members can comprise disc-shaped, conical shaped, tube-shaped, clover shaped and various other suitably shaped members.

In another aspect, the present invention comprises an implant for treating incontinence in a patient. The implant comprises a substantially thin, flexible sheet that has a geometry, size and shape suitable for implanting in the patient's retropubic space without extending through the patient's rectus fascia and without requiring the implant to be secured to substantially fixed anatomical structures such as the patient's pubic bone, periosteum of the pubic bone, Cooper's ligament and rectus fascia. Preferably, the sheet is capable of eliciting a foreign body response. Also preferably, the sheet comprises a synthetic mesh material having a plurality of holes, the holes being sized and shaped to afford tissue ingrowth to anchor the implant in the retropubic space. For example, woven and/or knitted polypropylene mesh materials are believed suitable.

Some patients have significant scarring in the retropubic space due to previous surgeries. In some instances the scarring can be so severe as to preclude the use of conventional sling procedures. The present invention is believed to be particularly suitable for an incontinent patient with scarring in the retropubic space as the surgeon need not significantly invade the suprapubic region.

These and other advantages of the invention are more fully shown and described in the drawings and detailed description of this invention, where like reference numerals are used to represent similar structures. It is to be understood, however, that the drawings and description are for the purposes of illustration only and should not be read in a manner that would unduly limit the scope of this invention.

### Brief Description of the Drawing

Other features and advantages of the present invention will be seen as the following description of particular embodiments progresses in conjunction with the drawings, in which:

Fig. 1 is a schematic view of selected female anatomy structures;

Fig. 1A is a schematic view of selected elements of the female anatomy at rest;

Fig. 1B is a schematic view of selected elements of a female anatomy during a stress event such as a cough;

Fig. 2 is a schematic view of an implant placed in a female according to the present invention;

Fig. 3. is a perspective view of a surgical instrument or inserter in accordance with an aspect of the present invention;

Fig. 4 is a schematic illustration of the surgical instrument of Fig. 3 used to place an implant in a female patient;

Fig. 5 is a side, partial section view of the surgical instrument of Fig. 3;

Fig. 6 is side view of another surgical instrument or inserter according to the present invention, after it has passed through endopelvic fascia, but prior to deploying an anchor;

Fig. 7 is a side view of the surgical instrument of Figure 6 after it has deployed an anchor;

Fig. 8 is a perspective view of an implant that is anchored to the endopelvic fascia with an anchor;

Fig. 9 is a perspective view of one embodiment of a deployable member or anchor according to one aspect of the present invention;

Fig. 10 is a sectional view of additional embodiments of surgical instrument and anchor according to the present invention, showing the anchor just being deployed relative to endopelvic fascia;

Fig. 11 shows the anchor of Fig 10 after it is fully deployed relative to the endopelvic fascia;

Fig. 12 is a top view of another anchor according to the present invention with arrows showing general directions in which components of the anchor may be folded;

Fig. 13 is a side view of the anchor of Fig. 12;

Fig. 14 is a top view of another anchor according to the present invention with arrows showing general directions in which components of the anchor may be folded;

Fig. 15 is a sectional view of another embodiment of surgical instrument according to the present invention;

Fig. 16 is a sectional view of additional embodiments of surgical instrument and anchor according to the present invention, showing the anchor prior to being deployed;

Fig. 17A is another side view of the invention shown in Figure 16, also showing a fully deployed anchor;

Fig. 17B shows the anchor of Fig. 17A after it is fully deployed;

Fig. 18 is a sectional view of additional embodiments of surgical instrument and anchor according to the present invention, showing the anchor prior to being deployed;

Fig. 19 is a sectional view of the embodiment of Fig. 18 after the anchor is deployed;

Fig. 20 is a side view of additional embodiments of surgical instrument and anchor according to the present invention, showing a rotary deployment anchor;

Fig. 21 is a side view of additional embodiments of surgical instrument and anchor according to the present invention,

Fig. 22 is a side view of additional embodiments of surgical instrument and anchor according to the present invention, showing an expanding tube anchor just prior to deployment;

Fig. 23 is a side view of another embodiment of anchoring structure according to an aspect of the present invention;

Fig. 24 is a side view of another embodiment of anchor according to the present invention, showing the anchor in a deployed position;

Fig. 25 is a bottom view of the anchor of Fig. 24;

Fig. 26 is a side view of the anchor of Fig. 24, showing the anchor in a pre-deployment position;

Fig. 27 is a top view of another embodiment of anchor according to the present invention;

Fig. 28 is a side view of another embodiment of deployable member, showing the member in a pre-deployment position;

Fig. 29 is a side view of the anchor of Fig. 28 in a deployed position;

Fig. 30 is a perspective view of an assembly for using a tissue adhesive according to an aspect of the present invention;

Fig. 31 is a schematic view of another embodiment of an implant placed relative to selected female anatomical structures according to the present invention; and

Fig. 32 is a perspective view of another embodiment of implant according to the present invention.

### Detailed Description

The following description is meant to be illustrative only and not limiting. Other embodiments of this invention will be apparent to those of ordinary skill in the art in view of this description.

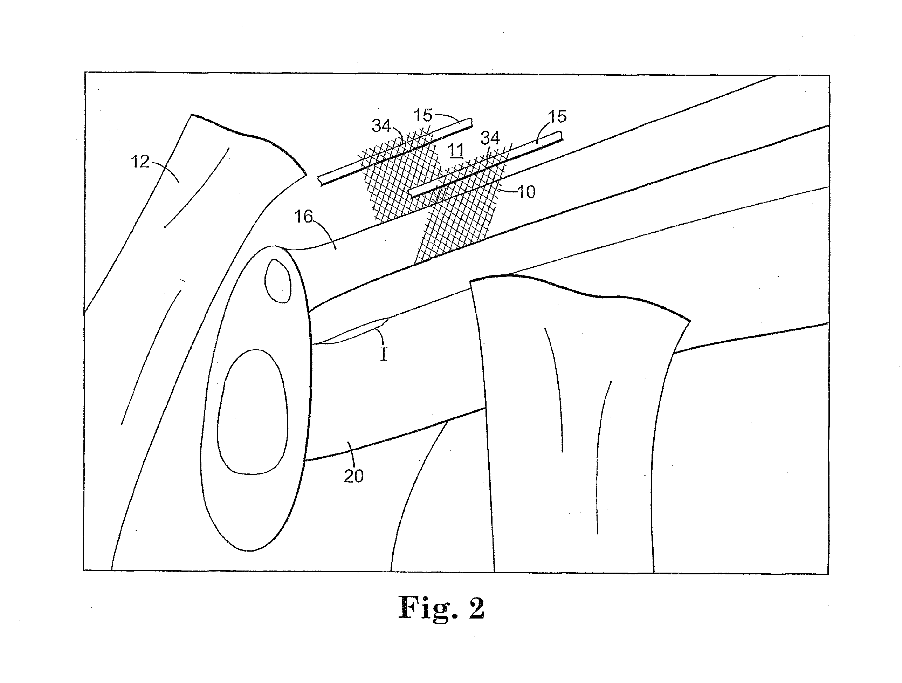

Referring to Figures 1 and 2, there is shown an implant 10 for treating incontinence in a patient. These figures schematically illustrate female anatomical features including the pubic bone 12, urethra 16, vagina 20, endopelvic fascia 15, a portion of the retropubic space 11, uterus 7, bladder 14, and rectus fascia 17. Notably, these structures are not shown to scale. For example, the retropubic space 11 is larger relative to other anatomical structures than the size depicted in Fig. 1.

The implant 10 comprises a thin, flexible structure that has a geometry, size and shape suitable for placement in the patient's retropubic space and for implantation in the retropubic space without bone anchors or suturing to Cooper's ligament or rectus fascia 17. In a preferred embodiment, the implant 10 is rectangular with a pair of sides and a pair of ends 34. Preferably, the implant 10 is adapted to be placed in the anatomical space above the endopelvic fascia 15 with minimum dissection and yet strengthen the area while providing at least a temporary fixation until healing has occurred.

The implant may be rectangular with a length of about less than ten inches (more preferably less than 5 or 4 inches) and a width of less than about 1 inch (more preferably between about 0.482 to 0.642 inches). While the implants are preferably rectangular for treating SUI in females, other shapes are also contemplated. Depending on the treatment addressed the implants may be any of a wide variety of shapes.

The present invention may be utilized in conjunction with a wide variety of implant materials. The implant may be integral, monolithic, or a composite of different components or segments of different components. Suitable non-synthetic materials include allografts, homo grafts, heterografts, autologous tissues, cadaveric fascia and fascia lata. Suitable synthetic materials for an implant include polymerics, and plastics and any combination of such materials. Commercial examples of such materials include Marlex™ (polypropylene), Prolene™ Mesh, polypropylene nonabsorbable synthetic surgical mesh available from Ethicon, of New Jersey, and Mersilene. Other examples of suitable materials include those disclosed in U.S. Patent Application Serial No. 09/939,098 filed August 24, 2001 (the entire contents of which are herein incorporated by reference). Specific examples of synthetic implant materials include, but are not limited to polypropylene, polyethylene, nylon, polyester (e.g. Dacron) PLLA and PGA. The implant material may be resorbable, absorbable or non-absorbable. Optionally, some portions may be absorbable and other portions may be non-absorbable.

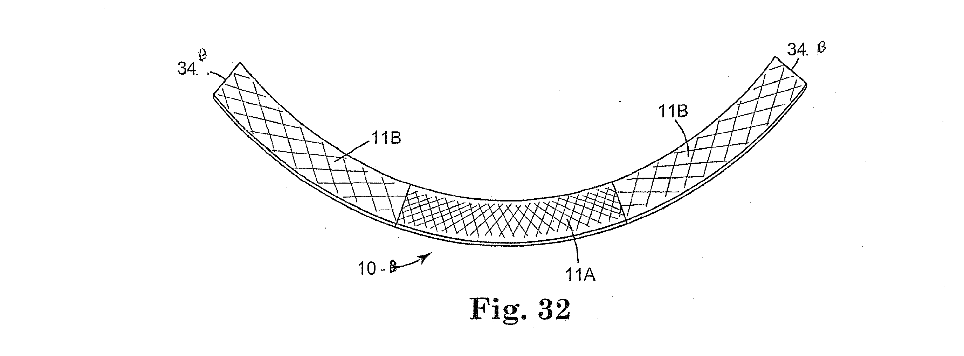

Figure 32 shows a sling 10B with ends 34B. The sling 10B has end portions 21B constructed of a different material than mid portion 11A. For example, the mid portion 11A may have a treatment that inhibits foreign body response to promote smooth integration of the portion of the sling most proximate the urethra. Alternatively, it can be constructed of a different material or weave to reduce tissue erosion.

In a preferred aspect of the invention, the implant may comprise a mesh material. The mesh material comprises one or more woven, knitted or inter-linked filaments or fibers that form multiple fiber junctions throughout the mesh. The fiber junctions may be formed via weaving, knitting, braiding, bonding, ultrasonic welding or other junction forming techniques, including combinations thereof. The size of the resultant openings or pores of the mesh are preferably sufficient to allow tissue in-growth and fixation within surrounding tissue.

Figure 2 illustrates an implant 10 with ends 34 projecting slightly through endopelvic fascia 15 and into endopelvic fascia (e.g. between 0.25 and 2 inches). The portion of the implant 10 near ends 34 preferably is initially loosely placed in the retropubic space but will afford anchoring over time due to the body's foreign body response. These portions of the sling 10 preferably have holes that are sized and shaped to encourage tissue ingrowth. This response will help anchor the implant 10 in a therapeutically effective position within the patient.

As an example, not intended to be limiting, the holes may comprise polygonal shaped holes with diagonals of 0.132 inches and 0.076 inches. The quantity and type of fiber junctions, fiber weave, pattern, and material type influence various implant properties or characteristics. As another example, not intended to be limiting, the mesh may be woven polypropylene monofilament, knitted with a warp tricot. The stitch count may be 27.5 courses/inch (+ or - 2 courses ) and 13 wales/inch (+ or - 2 wales). The thickness of this example is 0.024 inches. Non-mesh implant configurations are also included within the scope of the invention.

In another embodiment the implant material may have one or more substances associated therewith through a process such as coating or they may be incorporated into the raw material of the implant. Examples of appropriate substances include, without limitation, drugs, hormones, antibiotics, antimicrobial substances, dyes, silicone elastomers, polyurethanes, radiopaque filaments or substances, anti-bacterial substances, chemicals or agents, including any combinations thereof. The substances may be used to enhance treatment effects, reduce potential implant rejection by the body, elicit or inhibit a foreign body response, reduce the chances of tissue erosion, enhance visualization, indicate proper implant orientation, resist infection or other effects.

The sling 10 is preferably adapted to elicit a foreign body response. It is believed that an implant according to the present invention may be anchored in a predetermined position in the retropubic space even without external securing mechanisms (such as bone anchors or mechanical fasteners), particularly if sufficient time for tissue ingrowth is permitted. For example, the sling of Fig. 2 may be initially placed with absorbable sutures designed to last a predetermined amount of time (e.g. 1 to eight weeks), thereafter tissue reaction (e.g. ingrowth) may be relied upon to secure the sling 10 in place. The portion of the sling 10 projecting above the endopelvic fascia 15 is believed to be particularly useful in retaining the sling in position at that point.

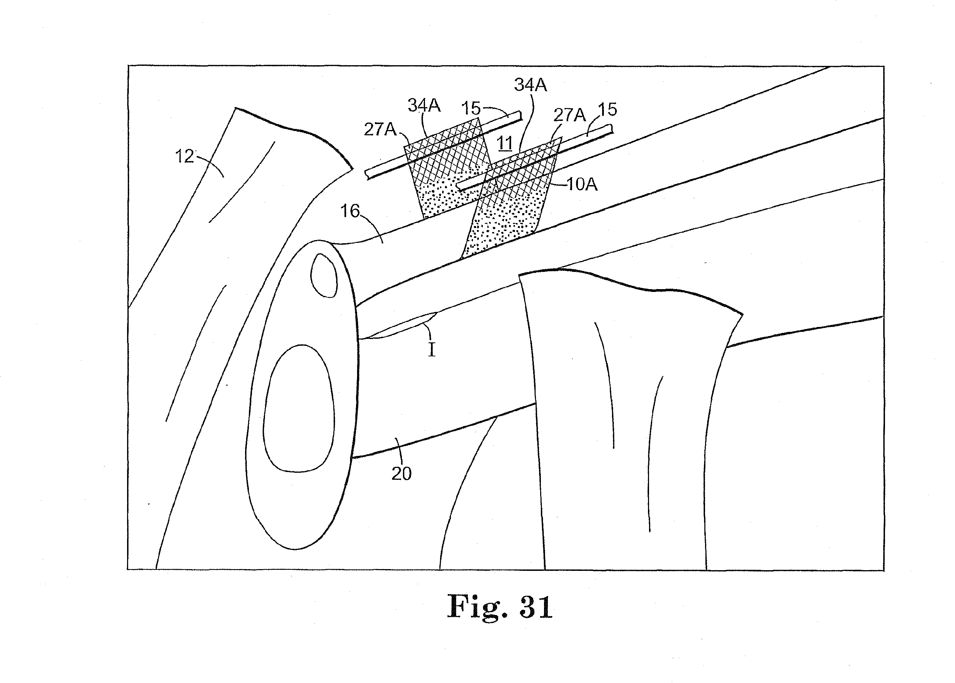

Figure 31 shows another embodiment of sling 10A according to the present invention. The sling 10A includes end portions 27A near ends 34A that are treated or constructed to elicit a foreign body response (e.g. promote scarring, or ingrowth) to afford secure anchoring of the sling 10A in the retropubic space and a middle region (designed to be place underneath urethra 16) that is designed to reduce the body's foreign body reaction and to avoid tissue damage (e.g. sling erosion).

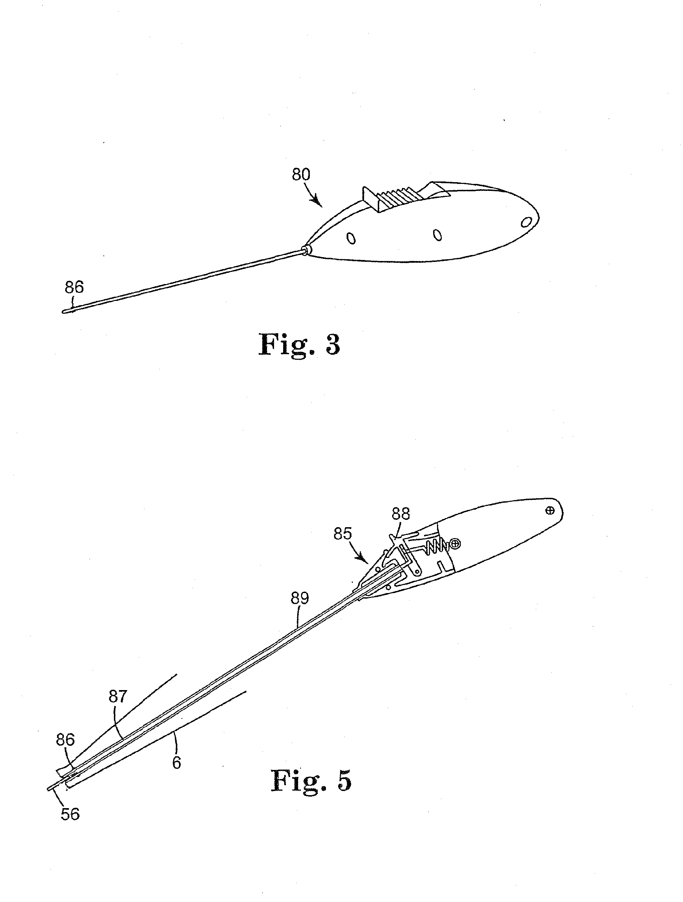

In a preferred embodiment, the present invention includes deployable members used to implant the implant 10 in the retropubic space 11.Referring to figures 3 through 5 and 9, there is shown deployable members 56. The deployable members 56 are particularly suitable for associating the implant 10 with endopelvic fascia 15 of the retropubic space 11. The deployable member 56 is preferably a nitinol wire formed in the shape of a cloverleaf (more preferably, four leaf). The anchor 56 can be folded and collapsed over itself to load it in an inserter or deployment tool (described below). When deployed, anchor 56 will preferably expand to 2-3 times the deployment tool diameter forming a rigid anchoring system.

The clover is wound to be flexible and thus able to collapse the 'leaves' of the clover in the plane of the clover. However, when deployed and expanded into its full state, it is very rigid in planes perpendicular to the 'leaves.' This property affords deployment of the anchor 56 with a tool that is smaller than the anchor yet, once the anchor 56 is deployed it will not collapse or pull out of tissue.

The deployable member 56 could be made from a flexible material such a Ni-Ti, Co-Cr-Ni-Mo-Fe, or other superelastic alloy. Polymers and plastics that are biocompatible long term are also contemplated for use to construct the member 56.

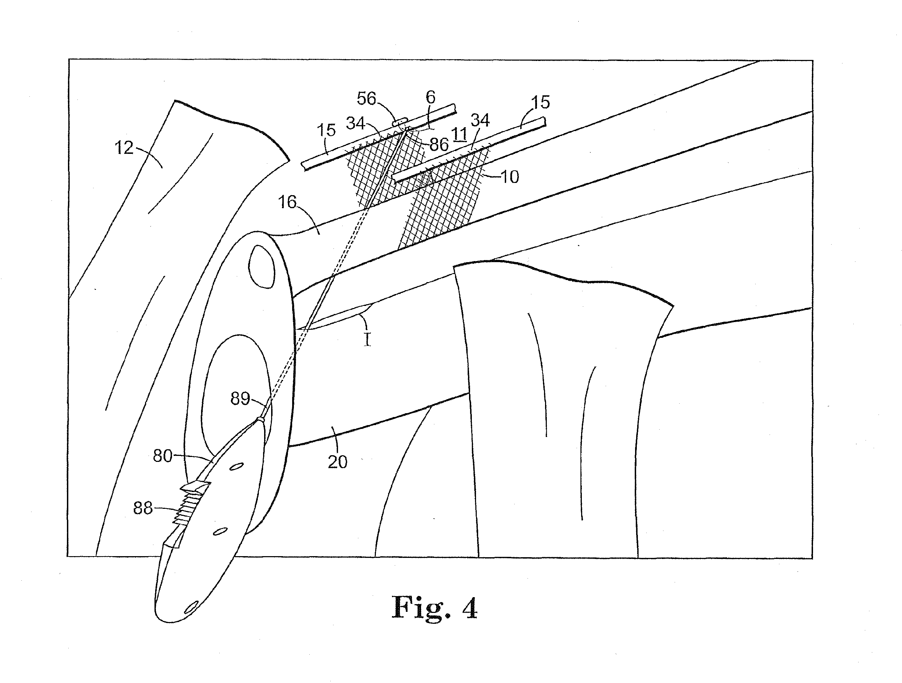

In another aspect, the present invention includes an inserter 80. As shown in Fig. 4, the inserter 80 is sized and shaped to associate the deployable members 56 with endopelvic fascia 15. The inserter 80 includes a sheath 89 with a distal end, and a movable member 87 within the sheath 89.

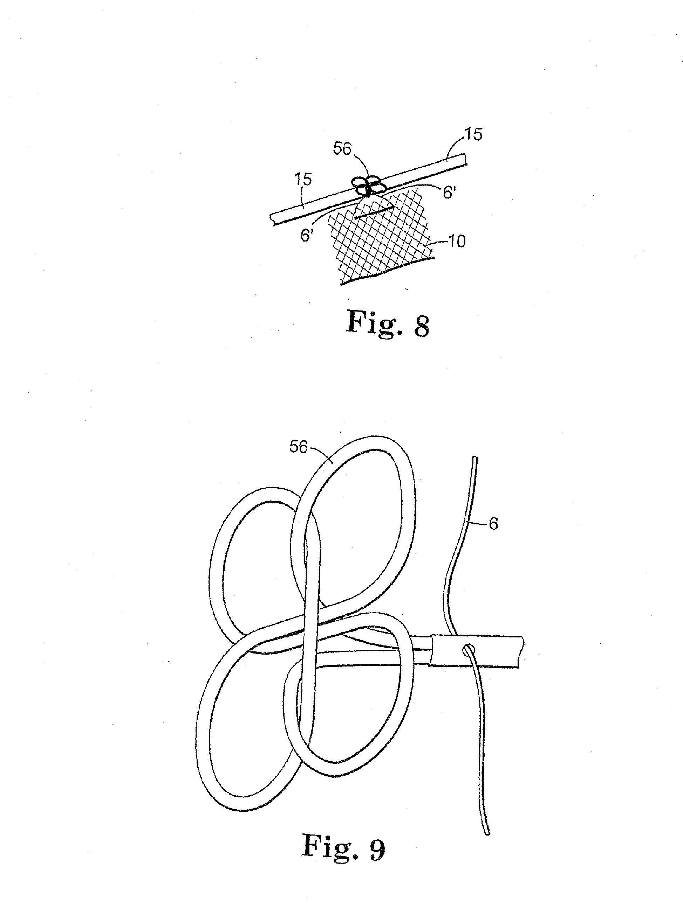

The movable member 87 is operatively associated with the deployable member 56 to move the deployable member between i) a retracted position with the deployable member 56 at least partially received within the sheath 89 of the inserter 80 (see Figure 5), and ii) an extended position spaced more distally to the distal end of the sheath 89 than in the retracted position. Button 88 affords linear movement of the movable member 87 so that it can push deployable member 56 out the distal end of sheath 89. Linear movement of the movable member 87 causes the deployable member 56 to move from the retracted position toward the extended position. Figure 8 shows the deployable member 56 after it is anchored in endopelvic fascia 15.

The deployable member 56 is capable of assuming a first orientation (Figure 5) that affords at least partial receipt of the deployable member within the sheath 89 of the inserter 80, and a second orientation (Figure 8) that affords association between the deployable member 56 and endopelvic fascia 15. In the depicted embodiment, the deployable member 56 comprises a substantially clover shaped top portion substantially situated in a first plane, and a stem substantially situated in a second plane. The stem includes a passage that anchors a suture 6. The suture 6 may then be used to tie a sling 10 to the endopelvic fascia (see Fig. 8).

The deployable member has a first profile in the first orientation (e.g. substantially flat in Fig. 5) and a second profile (e.g. substantially T-shaped as in Fig. 9) in the second orientation. In the first orientation, the first plane is nearly parallel to the second plane (i.e. the deployable member 56 is substantially flat), and in the second orientation, the first plane is substantially perpendicular to the second plane (i.e. the deployable member has a substantially T-shaped profile). The first profile is less than the second profile so that the deployable member 56 can fit in a sheath 89 that is smaller than the second profile.

The inserter 80 includes a tissue stop 86 for blocking insertion of the sheath 89 past preselected endopelvic fascia tissue 15. This helps prevent overinsertion of the sheath 89 into tissue, and the potential for damaging structures such as the bladder.

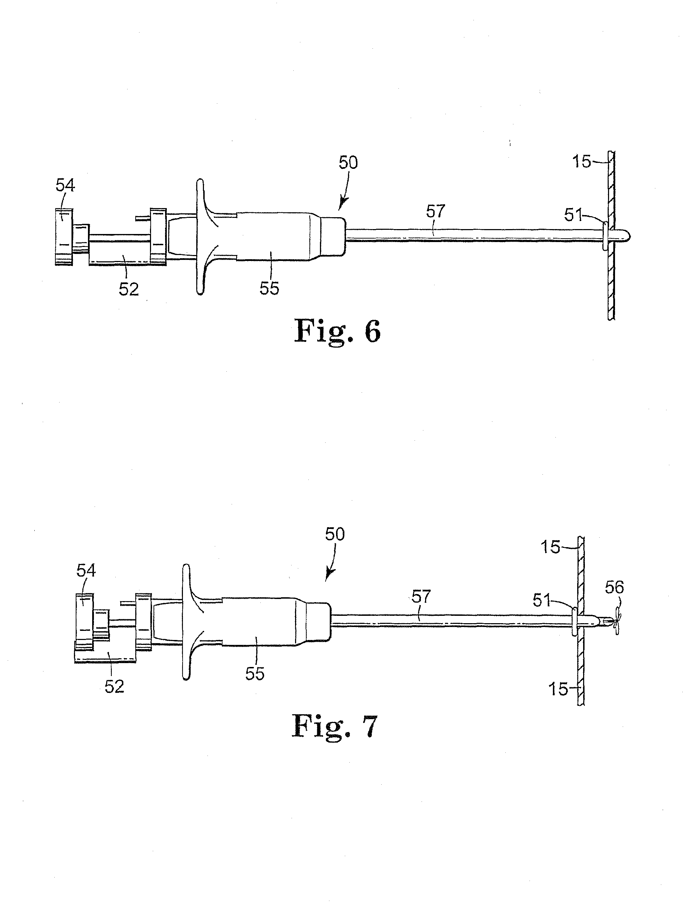

Figures 6 and 7 discloses another embodiment of inserter 50 according to the present invention. The inserter 50 includes a body 55 with finger flanges, sheath 57 with tissue stop 51, movable member 54 and lockout 52. Figure 6 shows the configuration of the elements of the inserter 50 as the distal end of the sheath 57 pierces endopelvic fascia 15. The lockout 52 blocks movement of the movable member 54 and prevents it from inadvertently moving forward (distally) prior to completely piercing the fascia 15. Once the distal end of the sheath 57 is placed in the predetermined position, the lockout 52 may be moved out of the path of the movable member 54 and the movable member may be used to eject the deployable member 56 from the distal end of the inserter 50.

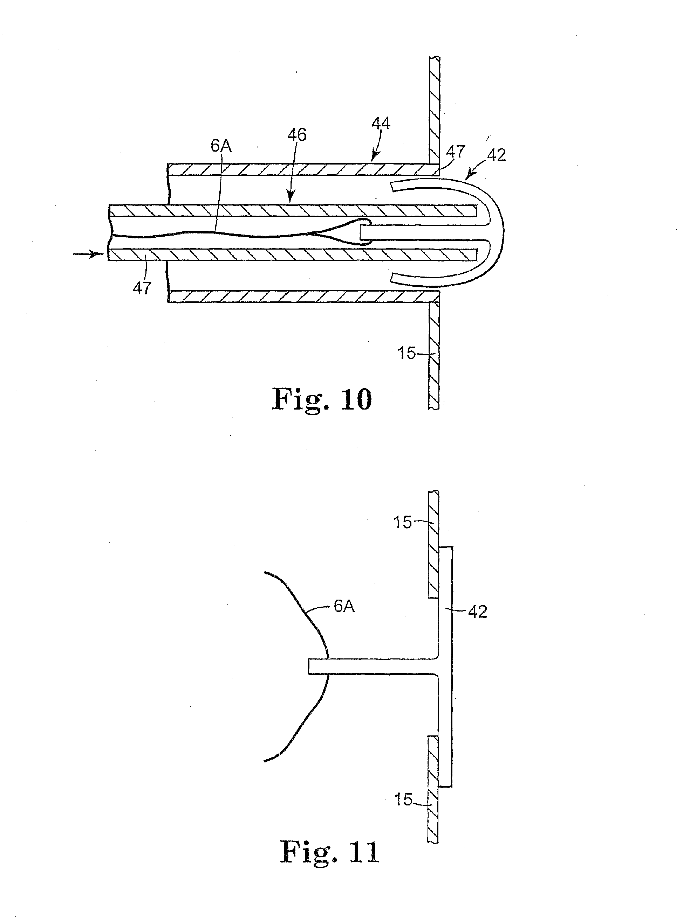

The deployable members according to the present invention may take several different forms. Figures 10 and 11 show a deployable member 42 that has a flexible, resilient, substantially disc shaped top portion, and a stem with an associated suture 6A. Fig. 10 also shows an inserter with a movable member 46 and sheath 44 relative to endopelvic fascia 15 just prior to deployment of deployable member 42. The movable member 46 has a hollow passage to receive the suture 6A. The passage helps manage the suture and prevent unwanted twisting or tangling of the suture. Figure 11 shows the deployable member 42 after it is ejected from the inserter by movable member 46. In this position, the deployable member 42 is free to resiliently deform to a configuration that readily anchors suture 6A.

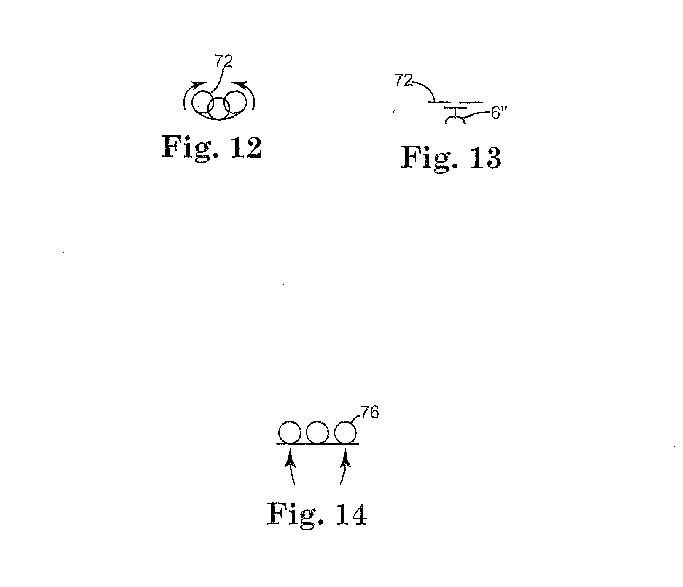

Figures 12 and 13 are top and side views of another embodiment of deployable member 72 according to the present invention. The deployable member 72 is resiliently deformable in the direction of the arrows in Figure 12 to a lower profile position to enable the member 72 to be received in an inserter device. Once the deployable member 72 passes through tissue, it can be deployed to anchor in tissue. Suture 6" is associated with the deployable member 72 so that a sling (e.g. 10) may be tied to member 72.

Three rings can be folded over on one another in various ways to fit in a smaller tube but will spring outward once deployed, thereby increasing surface area for anchoring. Three rings can be constructed from a single wire making three turns in it or making three rings and attaching them to a separate wire. From this perspective, the present invention can include an embodiment where a plurality of wire like structures are bound together such that, when they are advanced out of an inserter (e.g. 50 or 80), they spread out in a starburst fashion and form an anchor.

Figure 14 is a top view of another embodiment of deployable member 76 according to the present invention. The deployable member 76 is resiliently deformable in the direction of the arrows in Figure 14 to a lower profile position to enable the member 76 to be received in an inserter device.

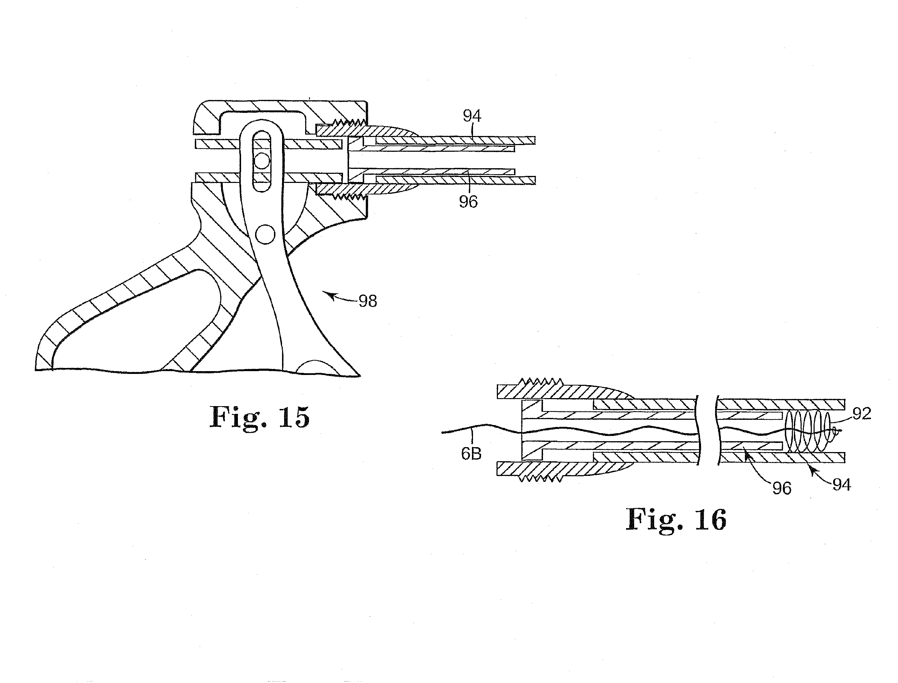

Figures 15 through 17b show another embodiment 90 of inserter 98 and deployable member 92 according to the present invention. The deployable member 92 comprises a resilient, helical or conical spring 92. A suture 6B is associated with the deployable member 92 (e.g. by being attached to the tip of the spring).

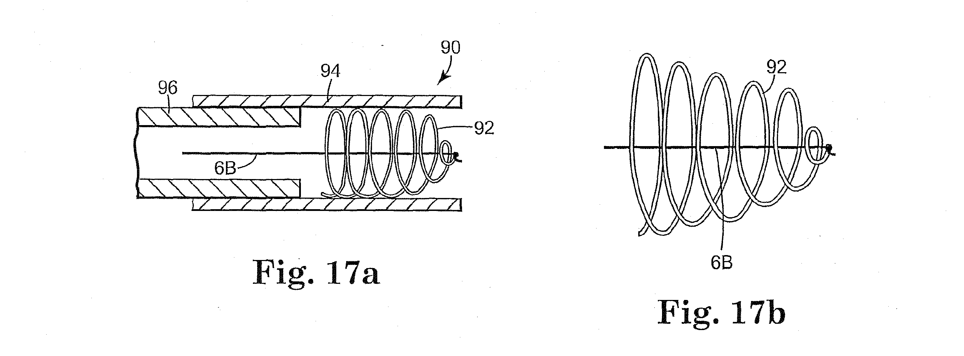

The inserter 98 includes a sheath 94 and a pusher 96. Optionally, the proximal portion of the inserter 98 could be constructed to be reusable, and the distal portion (e.g. including portions of the sheath 94 and a pusher 96) may be disposable. As shown in Fig. 17a, the deployable member 92 may be deformed to fit within sheath 94. After the pusher 96 pushes the deployable member 92 and suture 6B out the distal end of the sheath 94, the helical spring resiliently deforms to a shape (see Fig. 17b) suitable for anchoring in endopelvic fascia. Optionally, the spring 92 can be designed so that rotation of the spring 92 can afford adjustment of the sling tension (e.g. rotation in one direction tightens the sling, while rotation in the other direction loosens the sling).

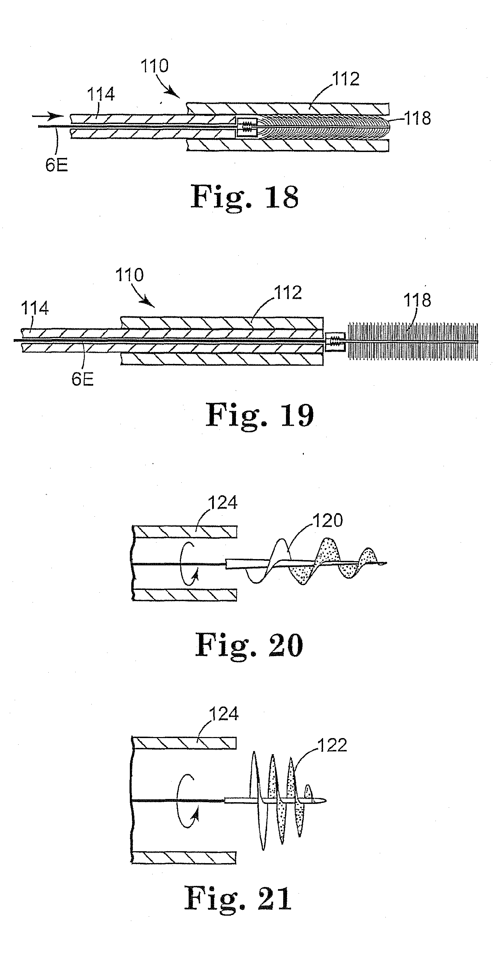

Figures 18 and 19 illustrate another embodiment of deployable member 118 and inserter 110 according to another aspect of the present invention. The deployable member 118 comprises a soft, brush shape with soft, resiliently flexible members or fingers. The brush shape dramatically increases the surface area of the deployable member for interaction with tissue to firmly anchor suture 6E in tissue. The suture 6B attaches to a base portion that can include a ratchet mechanism that affords adjustment of sling tension even after the suture 6E is tied to sling 10 (e.g. perioperative adjustment of the sling tension).

The inserter 110 includes an outer sheath 112 and a pusher member 114. The outer sheath 112 and member 114 are linearly movable relative to each other. Preferably, the sheath 112 retracts to deliver the deployable member so that the brush shaped deployable member 118 is not required to move through tissue.

Figures 20 and 21 illustrate additional embodiments of inserter and deployable members 120 and 122. The inserter includes an outer sheath 124. The deployable members 120 and 122 comprise screw-shaped anchor members. Preferably, the distal portion of the deployable member is constructed of a bioabsorbable material, while the portion of the deployable member that holds the suture in endopelvic fascia is constructed of a substantially permanent biocompatible material. In this embodiment, the movable member is rotatable in the direction of the arrow in the Figures.

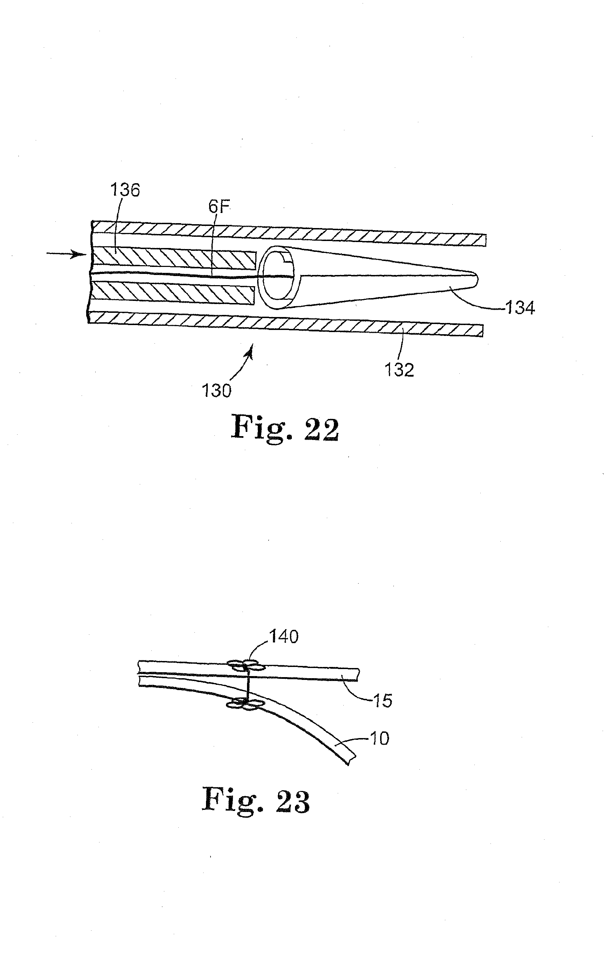

Figure 22 illustrates another embodiment of inserter 130 and deployable member 134 according to the present invention. The inserter 130 includes a sheath 132, and movable member 136. A suture 6F is associated with the deployable member 134. A rigid stem (not shown) attaches the suture 6F to the flexible deployable member 134.

As shown, the deployable member 134 comprises an expanding tube constructed from a biocompatible material. The expandable tube affords movement into tissue in one direction (e.g. deeper into endopelvic fascia), but resists movement though tissue in an opposite direction (e.g. out of endopelvic fascia). When the pusher 136 pushes on the rigid stem, the member 134 tends to take a smaller profile, thereby allowing the anchor to be placed deep in the endopelvic fascia 15. When the suture 6F is placed in axial tension (e.g. a pullout force), the tube 134 tends to expand to more firmly anchor in the tissue.

Figure 23 shows another embodiment of deployable member 140. The deployable member 140 includes two major surfaces. The two major surfaces allow endopelvic fascia 15 and an implant 10 to be situated therebetween. In one embodiment, the implant and tissue may be compressed between the major surfaces of the deployable member 140.

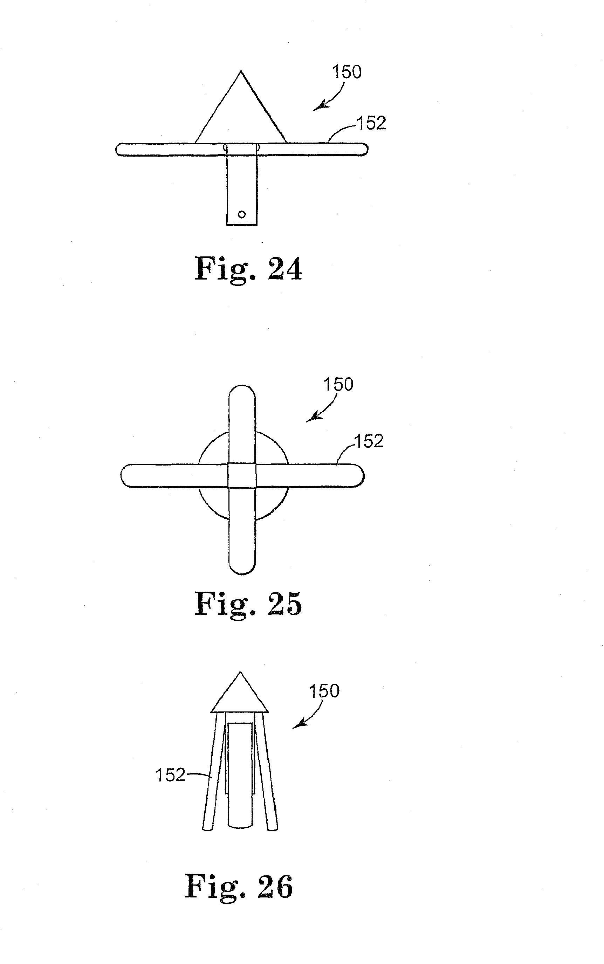

Figures 24 through 26 show another embodiment of deployable member 150 according to the present invention. The deployable member 150 includes a shaft, and a pointed tip to assist in piercing tissue. Preferably, this portion is constructed of a biocompatible, bioabsorbable material. The deployable member 150 also includes a plurality of movable arms 152. These elements are preferably constructed from a substantially permanent material (e.g. Delrin, Teflon or Nylon). The arms 152 may comprise living hinges associated with the shaft of the member 150.

Arms 152 could be in an extended position and bent down to load, thus springing back out when deployed. Alternatively, arms 152 could be made to be malleable, such that, upon deployment, the arms 152 are pushed out and are held in an outward position pursuant to plastic deformation. Arms 152 could be pinned and hang in a collapsed position and when deployed are pushed up and outward being held outward in an umbrella-like fashion.

Figures 24 and 25 show a configuration of the member 150 after it is deployed and suitable for use in anchoring a suture or implant in tissue such as endopelvic fascia.

Figure 26 shows a configuration of the member 150 adapted to be partially received in a shaft of an inserter device.

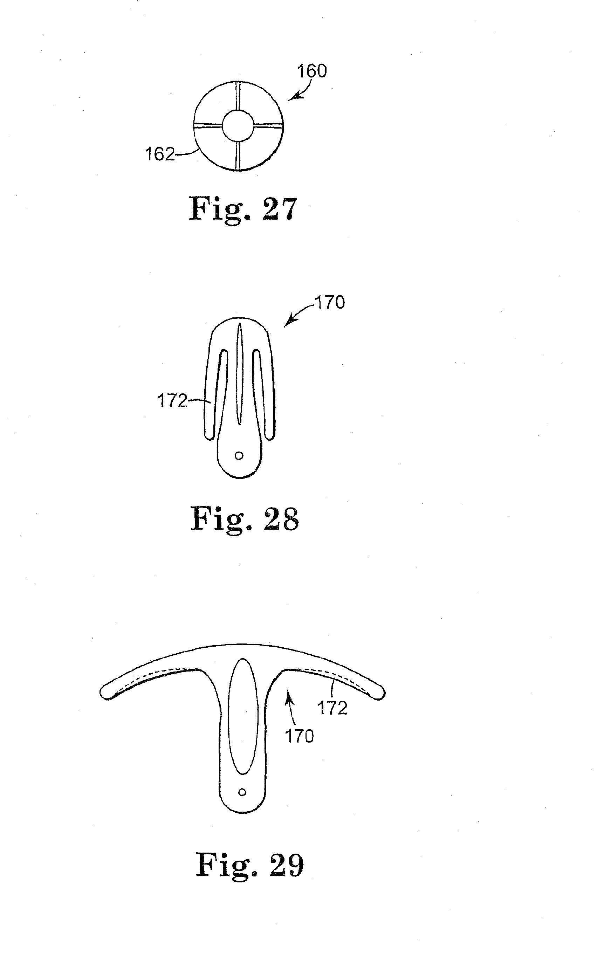

Figure 27 shows another embodiment of deployable member 160 according to the present invention. The deployable member 160 does not include a pointed tip. Instead, it includes a plurality of members 162 capable of resiliently expanding to form a substantially disc shaped top portion of the member 160.

Figures 28 and 29 show another embodiment of deployable member 170 according to the present invention. Again, the deployable member 170 does not include a pointed tip. The deployable member 170 includes spring fingers 172 adapted to resiliently expand after passing through endopelvic fascia. The deployable member 170 is particularly suitable for use with an inserter that has a sheath with a distal end suitable for piercing tissue, as the deployable member 170 does not include a point or sharp tip.

The deployable members of Figures 23 through 29 could be made from a flexible material such as Ni-Ti, Co-Cr-Ni-Mo-Fe, or other superelastic alloy. Also could use stainless steel or plastics for fabrication.

The implant 10 according to the present invention need not be anchored in the retropubic space with a mechanical fastener. For example, bioabsorbable sutures may be utilized to selectively hold the implant 10 in place during tissue ingrowth. The sutures should be designed to function long enough to afford sufficient ingrowth to anchor the implant 10 in the retropubic space.

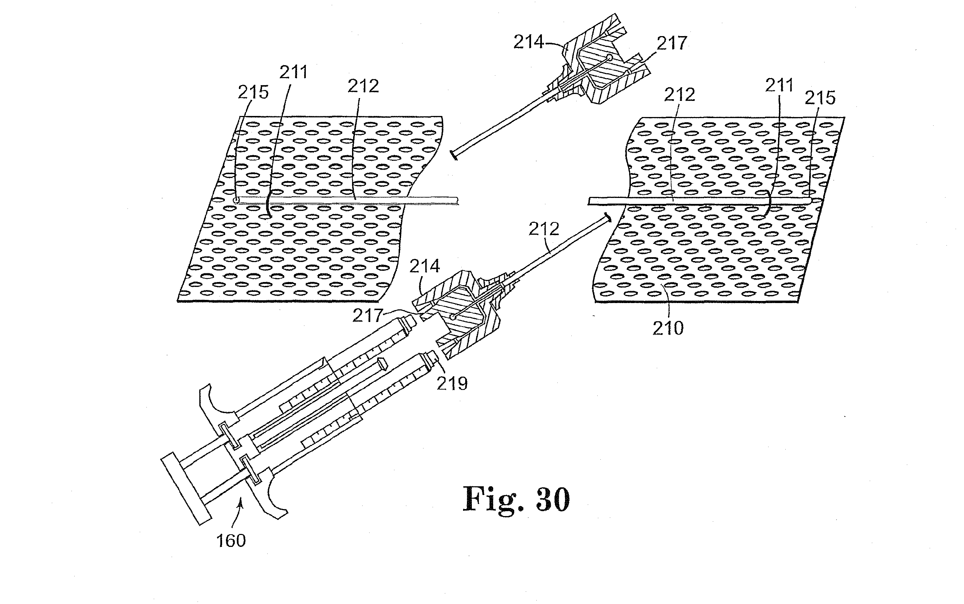

Figure 30 illustrates another embodiment of the present invention that does not utilize mechanical fasteners to anchor the implant 10 in the retropubic space. In this embodiment, the implant 10 is anchored by use of a tissue adhesive. Any suitable tissue adhesive may be utilized including those disclosed, for example, in U.S. Provisional Application Serial No. 60/279,794, filed March 29, 2001; U.S. Provisional Application Serial No. 60/302,929, filed July 3, 2001; U.S. Provisional Application Serial Number 60/307,836, filed July 25, 2001, and U.S. Provisional Application Serial No. 60/322,309, filed September 14, 2001 (the entire contents of each of which are herein incorporated by reference in their entirety).

Referring to Figure 30, a kit associated with this embodiment may include an implant 210, a syringe 160 and one or more tissue adhesive delivery needles 212 with ends 215 adapted to be associated with ends of the implant 210 (e.g. by loosely fitting, bioabsorbable sutures 211). The needles 212 may include a manifold 217 that is sealingly engageable with complementary surfaces 219 on the end of the syringe 160.

Since some tissue adhesives may include different storage requirements than the delivery components and/or implant 210, one preferred kit includes the implant 210, syringe 160 and delivery needles 212. The components of the tissue adhesive can be packaged separately and incorporated in the tubes of the syringe 160 just prior to use.

The delivery system optionally includes a means of attachment of the sling and transporting the sling into the retropubic space. After advancement of the adhesive/foam dispensing needle through the endopelvic fascia, an elastic, compressible foam or tissue adhesive may be dispensed. The foam or adhesive preferably spreads evenly into the fibrous material of the retropubic space, thereby affording sound anchoring. The even distribution of the adhesive or foam applies to a porous sling substance and ensures desirable integration with surrounding tissue.

In one embodiment, the tissue or foam may have a predetermined set time (e.g. 5-8 minutes) before hardening or becoming excessively tacky. This predetermined time may be used to adjust the tension of the sling underneath urethra 16. After satisfactory placement, needle 212 may be retracted and the sling 10 automatically disengages from the needle 212. The delivery tool may include release mechanisms, pushers or hooks to accomplish the disengagement.

The inserters and deployable members described above may be made from a variety of biocompatible and sterilizable materials including, without limitation, stainless steel, nitinol, acetal, Delrin®, Acrylonitrile-Butadiene-Styrene (ABS), polyethylene, nylon and any combination of materials.

In another aspect, the present invention comprises a kit for treating a patient (e.g. for SUI). The kit preferably comprises an inserter, an implantable material (e.g. implant) that is sized and shaped to be placed in the patient's retropubic space and at least two deployable members. Additional elements may also be included for surgical convenience, for avoidance of contamination from one portion of the body to another, for ease of manufacturing or sterilization, or for surgical requirements.

### Examples of Methods

Several methods are contemplated herein. Although the methods of use as disclosed herein generally relate to female incontinence conditions and treatments/procedures, male incontinence conditions and treatments/procedures are also included within the scope of the present invention. It should be noted that the present invention is particularly suitable for placing an implant in a therapeutically effective position. The method may be utilized to support a variety of structures at different anatomical locations. For example, the method may be used to correct mild to moderate fecal incontinence by correcting the patient's anal/rectal anatomical configuration. As such, the terms "space of Retzzus," "bladder", "urethro-vesical juncture", "vaginal vault", "urethra", "mid-urethra", "U-V juncture" and "bladder neck" are also included within the scope of the present invention.

Referring now to figure 4, a preferred embodiment of surgical procedure for treating female incontinence is disclosed according to an aspect of the present invention. Initially, the patient is placed under local, spinal or general anesthesia. A small transverse incision I is made in the anterior vaginal wall 20 of a female patient followed by minimal transurethral dissection.

An implant 10 is selected that is sized and shaped be implanted in the retropubic space. Notably, the implant 10 may be provided in a kit. The implant 10 may optionally be trimmed by the surgeon to address the particular needs of the surgical procedure (e.g. avoidance of scar tissue, or treating an individual with small anatomic features).

The patient is placed in a position suitable for a urological surgical procedure. Figure 4 simulates the position of anatomical features with a patient in the lithotomy position.

Figure 4 schematically illustrates one embodiment of the step of placing the implant 10 in the retropubic space 11 and in a therapeutically effective position relative to the patient's urethra 16 without extending the implant to the patient's rectus fascia (e.g. 17 in Fig. 1), without suturing the implant 10 to the patient's Cooper's ligament, and without using bone anchors to anchor the implant to the pubic bone 12. In this embodiment, inserter 80 is used to place a deployable member (e.g. 56) in endopelvic fascia (shown schematically as 15) of the patient.

Figure 4 shows a preferred embodiment where the step of providing an implant includes the step of providing an implant with first and second ends 34, and the step of implanting the implant includes the step of anchoring the first end of the implant with endopelvic fascia 15 on one side of the patient's urethra 16 and anchoring the second end 34 of the implant 10 with endopelvic fascia 15 on the other side of the patient's urethra 16. Four leaf clover shaped anchors (e.g. 56) are shown, but other fasteners could be used to anchor the implant in the retropubic space according to the present invention.

The implant is preferably placed mid-urethra as shown in Figure 4. However, it should be noted that other final locations are within the scope of the present invention, such as, placement of the implant 10 at the bladder neck.

Figure 4 shows an inserter 80 being used to pass a deployable anchoring member 56 with an associated suture 6 through endopelvic fascia 15. After the anchoring member 56 has substantially passed through the endopelvic fascia 15 (e.g. when stop 86 engages endopelvic fascia 15), the button 88 may be advanced to deploy the anchoring member 56.

The implant 10 is secured by tying the suture 6 to the implant 10. Figure 8 shows a suture 6' that is anchored in a step of member 56 and used to secure one end of the implant 10 to the anchor 56.

The steps described above are repeated as needed for a second side of the implant 10 on the other side of the urethra 16. As depicted, the step of implanting the implant 10 preferably includes the step of extending the implant 10 from the endopelvic fascia on one side of the patient's urethra 16, underneath approximately the mid-urethra, and to the endopelvic fascia 15 on the other side of the patient's urethra 16.

Other methods are also contemplated herein. For example, rather than using a mechanical fastener to anchor the implant 10, a tissue adhesive may be used to place the implant in the retropubic space. This embodiment offers the advantage that not even the endopelvic fascia 15 is pierced. Also, while the method preferably includes the step of creating a vaginal incision I, other surgical approaches are within the scope of the present invention including, for example, transurethral, laparoscopic and transperineal approaches (e.g. for treating male incontinence).

Although the invention has been described in terms of particular embodiments and applications, one of ordinary skill in the art, in light of this teaching, can generate additional embodiments and modifications without departing from the spirit of or exceeding the scope of the invention. Accordingly, it is to be understood that the drawings and descriptions herein are proffered by way of example to facilitate comprehension of the invention and should not be construed to limit the scope thereof.

## Claims

1. An assembly for placing an implant in a patient's retropubic space during a surgical procedure for treating incontinence, the assembly comprising:
at least one deployable member for associating the implant with endopelvic fascia of a retropubic space; and
an inserter that is sized and shaped to associate the deployable member with endopelvic fascia.

2. An assembly according to claim 1 wherein the deployable member is capable of assuming a first orientation that affords at least partial receipt of the deployable member within a sheath of the inserter, and a second orientation that affords secure association between the deployable member and endopelvic fascia.

3. An assembly according to claim 2 wherein the deployable member has a first profile in the first orientation and a second profile in the second orientation, and wherein the first profile is less than the second profile.

4. An assembly according to claim 1 wherein the deployable member comprises a flexible, substantially disc shape.

5. An assembly according to claim 1 wherein the inserter comprises a tissue adhesive dispenser and the deployable member comprises components of a tissue adhesive.

6. An assembly according to claim 2 wherein the deployable member comprises a substantially clover shaped top portion substantially situated in a first plane, and a stem substantially adapted to be situated in a second plane, and
in the first orientation, the first plane is nearly parallel to the second plane, and
in the second orientation, the first plane is substantially perpendicular to the second plane.

7. An assembly according to claim 1 wherein the deployable member comprises a conical spring.

8. An assembly according to claim 1 wherein the deployable member comprises a plurality of anchoring fingers.

9. An assembly according to claim 1 wherein the deployable member comprises an expandable tube that affords movement into tissue in one direction, but that resists movement through tissue in an opposite direction.
